# EUROPÄISCHE PATENTSCHRIFT

(11) **EP 0 944 323 B1**
(45) Veröffentlichungstag und Bekanntmachung des Hinweises auf die Patenterteilung: **12.03.2003**
(21) Anmeldenummer: 97952859.3
(22) Anmeldetag: 02.12.1997
(51) Int. Cl.: A01N 59/14, A01N 59/00, A61L 2/16

(54) **VERFAHREN ZUR INSTRUMENTENDESINFEKTION**
PROCESS FOR DISINFECTING INSTRUMENTS
PROCEDE POUR DESINFECTER DES INSTRUMENTS

(30) Priorität: 11.12.1996 DE 19651415
(43) Veröffentlichungstag der Anmeldung: 29.09.1999
(73) Patentinhaber: Ecolab GmbH & Co. OHG, 40589 Düsseldorf (DE)
(72) Erfinder: BIERING, Holger, D-41516 Grevenbroich (DE); BANSEMIR, Klaus-Peter, D-40764 Langenfeld (DE); SORNS, Jörg, D-40476 Düsseldorf (DE)
(86) Internationale Anmeldenummer: EP9706745
(87) Internationale Veröffentlichungsnummer: WO98025468

(56) Entgegenhaltungen:
- WO-A-95/02330
- WO-A-95/20876
- DE-A- 2 026 240
- GB-A- 2 268 879
- J. HOFMANN, G. JUST, W. PRITZKOW & H. SCHMIDT: "Bleaching activators and the mechanism of bleaching action." J. PRAKT. CHEM., Nr. 334, 1992, Seiten 293-297, XP002062685

## Beschreibung

Die vorliegende Erfindung betrifft die Verwendung eines Verfahrens zur Desinfektion von medizinischen Instrumenten zur Abtötung von Mykobakterien mit Hilfe wäßriger peroxidischer Zubereitungen.

Zur chemischen Desinfektion von Instrumenten mit Hilfe wäßriger Zubereitungen sind im Laufe der Zeit zahlreiche Vorschläge gemacht worden, in denen die unterschiedlichsten antimikrobiellen Wirkstoffe für die Desinfektion vorgesehen werden. In der Praxis haben Zubereitungen auf Basis von Aldehyden die weiteste Verbreitung gefunden, doch sind auch Präparate mit quartären Ammoniumverbindungen, Phenolen, Alkoholen und anderen Desinfektionswirkstoffen in Gebrauch. Zubereitungen auf Basis peroxidischer Wirkstoffe, insbesondere von Peressigsäure haben dagegen für diese Anwendung nur geringe Bedeutung erlangt. Ein wesentlicher Grund liegt in der geringen Lagerstabilität derartiger wäßriger Zubereitungen. Wegen der breiten antimikrobiellen Wirksamkeit der Peroxide hat es nicht an Versuchen gefehlt, den Nachteil der geringen Lagerstabilität zu überwinden. So ist beispielsweise in den deutschen Offenlegungsschriften 26 55 599 und 28 15 400 vorgeschlagen worden, die für die Desinfektion benötigten wäßrigen Zubereitungen erst kurz vor Gebrauch aus stabileren Vorstufen, nämlich aus Natriumperborat und Säureanhydriden, herzustellen. Gemäß der deutschen Offenlegungsschrift 27 01 133 werden die wäßrigen Zubereitungen aus Wasserstoffperoxid-Abspaltern und aromatischen Acyloxycarbonsäuren erhalten. Nur wenige dieser Verbindungen liefern aber Desinfektionslösungen mit ausreichend breiter Wirksamkeit, und die Lagerung dieser Acylierungsmittel im Gemisch mit den nötigen anorganischen Peroxiden ist wegen Zersetzungsreaktionen auch nur begrenzte Zeit möglich. Unter der Bezeichnung Sekusept Pulver ist ein Produkt im Handel, bei dessen Auflösung in Wasser durch Umsetzung von Natriumperborat mit Tetraacetylethylendiamin (TAED) eine desinfektionswirksame Zubereitung entsteht. Dieses Produkt auf Basis einer N-Acylverbindung besitzt ein breites Wirkungsspektrum und ist lagerstabil. Obwohl auf diese Weise bereits ein hoher Standard bei der Desinfektion von medizinischen Instrumenten erreicht worden ist, wurde weiter an der Verbesserung der peroxidischen Systeme gearbeitet, um noch bestehende Wirkungslücken und Nachteile im Gebrauch zu beseitigen. So ist beispielsweise in der DE-OS 36 15 787 vorgeschlagen worden, anstelle von anorganischen Wasserstoffperoxid-Abspaltern das Magnesiumsalz der Monoperoxiphthalsäure bei der Herstellung derartiger Zubereitungen zu verwenden. Der Einsatz dieses organischen Peroxids bedeutet jedoch gegenüber dem Einsatz der lagerstabilen und preiswerten anorganischen Peroxide einen erheblich höheren technischen Aufwand. Aufgabe der vorliegenden Erfindung war es daher, eine Verbesserung der Desinfektionssysteme auf Basis anorganischer Peroxide und N-Acylverbindungen zu erreichen. Eine der Detailaufgaben war es dabei, ein Desinfektionsverfahren zu entwickeln, das es erlaubt, auch Mykobakterien sicher zu desinfizieren. Weiterhin sollten leichte Handhabbarkeit, gute Lagerstabilität und geringe Korrosionsneigung gewährleistet werden.

In der WO-A-95/02330 wird ein putverförmiges Desinfektionsmittel beschrieben, welches Perborat, ein schnell-wirksames Acetylierungsmittel, beispielsweise TAED, ein langsam-wirksames Acetylierungsmittel, beispielsweise Acetylsalicylsäure, und eine Puffersubstanz, beispielsweise Phosphate, enthält. Beim Auflösen des Mittels in Wasser - unter Bildung von Persessigsäure - bewirkt die Puffersubstanz eine Einstellung des pH-Wertes der gebildeten Lösung auf Werte im Bereich von 7,5 bis 9. Auf diese Weise wird eine verbesserte Stabilität der gebildeten Peressigsäure-Lösung erzielt. Bevorzugt ist es, den pH-Wert auf Werte im Bereich von 7,5 bis 8,5 einzustellen. In dieser Form wird die gebildete Lösung zu Desinfektionszwecken eingesetzt.

Die DE-A-20 26 240 offenbart ein Verfahren zur Desinfektion medizinischer Geräte und Instrumente durch Behandeln derselben mit einer wäßrigen Lösung eines Desinfektionsmittels. Diese Lösungen werden erhalten durch Umsetzung einer in Wasser Wasserstoffperoxid liefernden Aktivsauerstoffverbindung mit einem Aktivator in Form einer Acyl-Verbindung in wäßriger Lösung bei einem alkalischen pH-Wert im Bereich von 7 bis 12, vorzugsweise 7,5 bis 11 und insbesondere von 8 bis 11. Die gebildeten Lösungen werden in dieser Form zur Desinfektion eingesetzt.

Es wurde jetzt gefunden, daß eine wesentliche Verbesserung der bisher bekannten Desinfektionssysteme auf Basis anorganischer Wasserstoffperoxid-Abspalter und N-Acylverbindungen durch eine verblüffend einfache Maßnahme erreicht werden kann.

Gegenstand der vorliegenden Erfindung ist die Verwendung eines Desinfektions-Verfahrens für medizinische Instrumente zur Abtötung von Mykobakterien durch Behandeln der Instrumente mit einer mikrobizid wirkenden wäßrigen Zubereitung, dadurch gekennzeichnet, daß bei der Herstellung dieser Zubereitung folgende zwei Schritte A und B durchlaufen werden:
A: Umsetzen von Wasserstoffperoxid oder von peroxidischen Verbindungen, die in Wasser Wasserstoffperoxid bilden, in wäßrig-alkalischem Medium bei einem pH-Wert im Bereich von 9 bis 11 mit N-Acylverbindungen, die unter diesen Bedingungen in der Lage sind, Wasserstoffperoxid zu acylieren,
B: Absenken des pH-Wertes der in Schritt A erhaltenen Zubereitung und gegebenenfalls Verdünnung, so daß in der zur Desinfektion eingesetzten Zubereitung ein pH-Wert zwischen 7 und 9 vorliegt.

Durch die vergleichsweise einfache Maßnahme der nachträglichen Absenkung des pH-Wertes, die mit üblichen Säuren vorgenommen werden kann, wird eine außergewöhnliche Steigerung der antimikrobiellen Wirksamkeit bei gleichzeitig sehr geringer Korrosionsneigung erreicht, ohne daß die Vorteile des bisherigen Verfahrens aufgegeben würden.

Bei der Herstellung der mikrobizid wirkenden wäßrigen Zubereitung wird im erfindungsgemäßen zu verwendenden Verfahren in Schritt A von Wasserstoffperoxid oder von solchen peroxidischen Verbindungen ausgegangen, die beim Auflösen in Wasser unmittelbar Wasserstoffperoxid freisetzen. Bei diesen peroxidischen Verbindungen kann es sich um Addukte des Wasserstoffperoxids an verschiedene Träger, die gelegentlich auch als Perhydrate bezeichnet werden, handeln, beispielsweise Harnstoff-Perhydrat, Natriumcitrat-Perhydrat oder Natriumcarbonatperhydrat (Na₂CO₃ x 1,5 H₂O₂), das üblicherweise auch als Natriumpercarbonat bezeichnet wird. Daneben eignen sich echte anorganische Peroxoverbindungen, die in Wasser spontan hydrolisieren, wie beispielsweise die Natriumperborate, beispielsweise Natriumperboratmonohydrat und Natriumperborattetrahydrat. Nicht geeignet sind dagegen organische Peroxoverbindungen, in denen die Peroxogruppe - direkt an Kohlenstoff gebunden ist. Besonders bevorzugt werden im Rahmen des erfindungsgemäß zu verwendenden Verfahrens Natriumpercarbonat sowie das Mono- und Tetrahydrat des Natriumperborats, von denen wiederum das Monohydrat besonders bevorzugt wird. Es können auch mehrere Perverbindungen gleichzeitig eingesetzt werden.

Als N-Acylverbindungen kommen für die Umsetzung in Schritt A prinzipiell alle Verbindungen dieser Gruppe in Frage, die auch im Bereich der Waschmittelchemie als sogenannte Bleichaktivatoren zur Umsetzung mit Wasserstoffperoxid in alkalischen Waschflotten beschrieben worden sind. Geeignete N-Acylverbindungen sind insbesondere diejenigen, die an dem Stickstoff, der die Acylgruppe trägt, eine weitere Ketogruppe aufweisen, und/oder in denen der Stickstoff Teil eines heterocyclischen Ringsystems ist. Beispiele geeigneter N-Acylverbindungen sind die mehrfach acylierten Alkylendiamine, wie etwa Tetraacetylethylendiamin, acylierte Glykolurile, in erster Linie Tetraacetylglykoluril, N-acylierte Hydantoine, Hydrazide, Triazole, Triazine, Urazole, Diketopiperazine, Sulfurylamide, Lactame und Cyanurate. Im Rahmen des erfindungsgemäß zu verwendenden Verfahrens werden Tetraacetylethylendiamin (TAED), Tetraacetylglykoluril (TAGU) und 1,5-Diacetyl-2,4-dioxohexahydro-1,3,5-triazin (DADHT) bevorzugt, von denen wiederum Tetraacetylethylendiamin ganz besonders bevorzugt wird. Selbstverständlich können auch mehrere N-Acylverbindungen gleichzeitig eingesetzt werden.

Die Umsetzung der Wasserstoffperoxid liefernden Verbindung und der N-Acylverbindung in Schritt A wird in der Weise vorgenommen, daß beide Verbindungen nacheinander oder, vorzugsweise, gleichzeitig in Wasser eingebracht werden. Falls die peroxidische Verbindung, wie etwa Natriumpercarbonat und Natriumperborat, eine ausreichende Alkalität zur Verfügung stellen, kann dabei auf den Zusatz von Alkalisierungsmitteln verzichtet werden. Andernfalls müssen alkalisch reagierende Substanzen, vorzugsweise alkalisch reagierende anorganische Salze, wie etwa Natriumcarbonat, Alkaliphosphate oder Alkaliborate, zugesetzt werden, um einen pH-Wert einzustellen, der auch nach Abschluß der Umsetzung noch im alkalischen Bereich zwischen 9 und 11 liegt. Das Verhältnis von Wasserstoffperoxid liefernder Verbindung und N-Acylverbindung wird vorzugsweise so gewählt, daß pro Mol reaktiver Acylgruppen in der N-Acylverbindung 0,5 bis 10 Mol, vorzugsweise 1 bis 5 Mol Wasserstoffperoxid zur Verfügung stehen. Vorzugsweise werden daher etwa 0,1 bis etwa 1 Gew.-%, insbesondere etwa 0,2 bis etwa 0,6 Gew.-% an peroxidischer Verbindungen und etwa 0,1 bis etwa 1 Gew.-%, insbesondere etwa 0,2 bis etwa 0,5 Gew.-% an N-Acylverbindung, bezogen auf das Gesamtgewicht der Zubereitung in Schritt A, eingesetzt.

Die Umsetzung zwischen Wasserstoffperoxid und der N-Acylverbindung in Schritt A ist eine Zeitreaktion, die von der Konzentration der Reaktionspartner, der Reaktivität der N-Acylverbindung, dem pH-Wert der Lösung und von der Temperatur abhängt. Die Reaktion kann durch Erhöhung der Konzentrationen und durch Erhöhung der Temperatur beschleunigt werden. Vorzugsweise wird die Reaktion im Temperaturbereich zwischen etwa 10 und etwa 45 °C durchgeführt, wobei insbesondere Temperaturen im Bereich der Raumtemperatur und wenig darüber gewählt werden. Mit besonders geeigneten N-Acylverbindungen verläuft die Umsetzung so schnell, daß unter den bevorzugten Bedingungen innerhalb von 1 bis 20 Minuten, insbesondere innerhalb von 2 bis 10 Minuten wenigstens 50 % der N-Acylverbindung abreagiert haben:

Die Absenkung des pH-Werts gemäß Schritt B wird vorzugsweise erst dann eingeleitet, wenn die N-Acylverbindung in Schritt A zu mehr als 50 %, insbesondere zu mehr als 70 % und besonders bevorzugt zu mehr als 80 % abreagiert hat. Die Absenkung des pH-Werts wird durch Zusatz sauer reagierender Stoffe, insbesondere anorganischer oder organischer Säuren oder sauer reagierender Salze in geeigneter Menge vorgenommen. Weiterhin kann in diesem Schritt B, falls nötig, eine Verdünnung erfolgen, so daß die in Schritt A gebildeten peroxidische Wirkstoffe die für den Desinfektionsvorgang richtige Konzentration erreichen. Die Menge an Säure bzw. sauer reagierender Verbindung wird so gewählt, daß in der für die Desinfektionsanwendung vorgesehenen Lösung ein pH-Wert im Bereich von 7 bis 9, insbesondere im Bereich 7,5 bis 8,5 erreicht wird. Besonders geeignete Säuren für die Absenkung des pH-Wertes in Schritt B sind beispielsweise Phosphorsäure, Essigsäure, Citronensäure und allgemein wasserlösliche organische Säuren. Als geeignete sauer reagierende Salze seien beispielsweise saure Phosphate wie NaH₂PO₄ und Hydrogensulfate genannt. Besonders bevorzugt werden in Schritt B des erfindungsgemäß zu verwendenden Verfahrens Phosphorsäure, Essigsäure und Citronensäure verwendet, von denen wiederum Phosphorsäure besonders bevorzugt wird.

In Schritt A und/oder Schritt B können der Zubereitung weitere Hilfs- und Zusatzstoffe beigefügt werden, wenn dies für die erfindungsgemäße Verwendung zur Desinfektion von medizinischen Instrumenten vorteilhaft ist. In Schritt A kommen dabei als weitere Hilfsstoffe vorzugsweise Tenside, Alkalisierungsmittel, Komplexbildner für Wasserhärte, Komplexbildner für Schwermetallionen und wasserlösliche anorganische Salze in Betracht. In Schritt B kommen als Hilfsstoffe vorzugsweise Korrosionsinhibitoren und Tenside in Betracht. Die Menge an derartigen Hilfsstoffen kann in den Zubereitungen in sehr weiten Grenzen, abhängig von der beabsichtigten Wirkung, schwanken. Sie liegt üblicherweise nicht über etwa 3 Gew.-%. vorzugsweise zwischen etwa 0,001 und etwa 1 Gew.-%, bezogen auf die gesamte Zubereitung.

Als Tenside eignen sich sowohl anionische als auch nichtionische Tenside, ferner können auch katipnische und amphotere Tenside in Betracht kommen. Bevorzugt werden als Tenside in Schritt A anionische Tenside und nichtionische Tenside sowie gegebenenfalls Mischungen mehrerer Tenside aus diesen beiden Klassen. Als geeignete anionische Tenside sind besonders zu erwähnen: Alkylbenzolsulfonate, Alkylsulfate, das sind die Salze von Schwefelsäurehalbestem langkettiger Alkohole, Alkylethersulfate, das sind Salze von Schwefelsäurehalbestem langkettiger alkoxylierter, insbesondere ethoxylierter Alkohole, Alkansulfonate und Olefinsulfonate. Die anionischen Tenside werden vorzugsweise als Natriumsalze verwendet. Als bevorzugt geeignete nichtionische Tenside seien die alkoxylierten langkettigen Alkohole erwähnt. Bevorzugt werden die mit Ethylenoxid alkoxylierten Alkohole und die mit Ethylenoxid sowie einer geringen Menge Propylenoxid alkoxylierten Typen. Als weitere bevorzugte nichtionische Tenside, die insbesondere dann eingesetzt werden, wenn es auf Schaumarmut ankommt, sind die sogenannten endgruppenverschlossenen Alkoxylierungsprodukte zu erwähnen, die aus den vorgenannten nichtionischen Tensiden durch Veretherung der endständigen Hydroxylgruppe mit kurzkettigen Alkoholen zugänglich sind.

In Schritt B können prinzipiell dieselben Tenside eingesetzt werden, wie sie für den Schritt A beschrieben wurden, doch werden hier die nichtionischen Tenside stärker bevorzugt. Sofern die Tenside gemeinsam mit den zur Absenkung des pH-Wertes in Schritt B benötigten Säuren konfektioniert werden, kommen für diese Anbietungsform besonders die säurestabilen Tenside in Betracht.

Als Komplexbildner für die Wasserhärte ist in erster Linie Natriumtriphosphat zu erwähnen, doch kommen hierfür auch andere Polyphosphate, Salze der Nitrilotriessigsäure und Salze von organischen Polycarbonsäuren, beispielsweise Citronensäure, oder von polymeren Polycarbonsäuren, beispielsweise Acrylsäure-Maleinsäure-Copolymerisaten, in Betracht. Besonders bevorzugt wird Natriumtriphosphat, das zugleich als Alkalisierungsmittel wirkt.

Als Komplexbildner für Schwermetallionen, die zersetzend auf peroxidische Verbindungen wirken, kommen in erster Linie Aminopolycarbonsäuren bzw. deren Salze, beispielsweise Ethylendiamintetraessigsäure, insbesondere aber Aminopolyphosphonsäuren, wie Ethylendiamintetramethylenphosphonsäure, oder auch Hydroxyethandiphosphonsäure bzw. deren Salze in Betracht.

Wasserlösliche Salze können die Funktion von Füllstoffen oder Gerüststoffen übemehmen, wie beispielsweise Natriumsulfat, sofern sie nicht gleichzeitig alkalisierende Wirkung haben, wie beispielsweise Natriumcarbonat und Natriumsilikat. Als Korrosionsinhibitoren sind insbesondere Alkylphosphonsäuren zu erwähnen, von denen Octanphosphonsäure besonders bevorzugt wird. Als weitere mögliche Hilfsstoffe sind Farbstoffe, Parfüm und lösungsvermittelnde Zusätze zu erwähnen.

Zur einfachen Durchführung des erfindungsgemäß zu verwendenden Verfahrens kann es zweckmäßig sein, sämtliche in den Schritten A bzw. B einzusetzenden Substanzen in jeweils einem Produkt in der gewünschten Menge zu vereinigen, so daß sich die Durchführung der Schritte A und B in einfacher Weise dadurch vornehmen läßt, daß die jeweiligen Produkte in der notwendigen Menge Wasser gelöst werden (Schritt A) bzw. der in Schritt A entstandenen Zubereitung nach vorgegebener Zeit zugesetzt werden (Schritt B). Diese für die Durchführung der Schritte A bzw. B vorgefertigten Produkte können fest oder flüssig sein, je nach Aggregatszustand der einzusetzenden Substanzen. Vorzugsweise handelt es sich bei dem für Schritt A vorgesehenen Produkt um eine pulverförmige Mischung der Einzelsubstanzen, wobei zweckmäßigerweise die Partikelform so gewählt wird, daß sich die einzelnen Substanzen ausreichend schnell lösen und damit für die Umsetzung zur Verfügung stehen. Für die Lagerstabilität des pulverförmigen Produktes selbst kann es wiederum zweckmäßig sein, einzelne oder mehrere der Substanzen, insbesondere die N-Acylverbindungen und/oder die peroxidische Verbindung in granulierter Form oder in umhüllter Form zu verwenden. Anstelle eines Pulvers kann das für Schritt A vorgesehene Produkt aber auch in kompakterer Form, beispielsweise als Tablette angeboten werden, sofern beispielsweise durch Zugabe geeigneter Sprengmittel sichergestellt ist, daß diese Tabletten sich in Wasser ausreichend schnell lösen.

Eine feste Anbietungsform ist auch bei dem für Schritt B vorgesehenen Produkt möglich, doch kann hier wegen der geringeren Stabilitätsprobleme während der Lagerung ebensogut eine flüssige Anbietungsform gewählt werden. Insbesondere kommen hier konzentrierte wäßrige Lösungen in Betracht. Auch bei diesem Produkt kann es vorteilhaft sein, es in portionierter Form anzubieten, um beispielsweise die Einhaltung von Dosierungsvorgaben zu erleichtern.

Ein für die Durchführung von Schritt A durch Auflösen in Wasser bestimmtes Produkt kann insbesondere die folgende Zusammensetzung haben:
5 bis 40 Gew.-%, vorzugsweise 10 bis 30 Gew.-% feste anorganische Perverbindung,
5 bis 30 Gew.-%, vorzugsweise 10 bis 20 Gew.-% pulverförmiges TAED,
20 bis 50 Gew.-%, vorzugsweise 30 bis 45 Gew.-% Natriumtriphosphat
0 bis 15 Gew.-%, vorzugsweise 1 bis 10 Gew.-% Tensid und
zu 100 Gew.-% lösliches anorganisches Salz und ggf. weitere Hilfsstoffe
Es wird vorzugsweise in einer Menge von 1 bis 10 Gew.-% in Wasser gelöst.

Ein als Zusatz in Schritt B geeignetes Produkt kann insbesondere folgende Zusammensetzung haben:
40 bis 80 Gew.-%, vorzugsweise 55 bis 65 Gew.-% konzentrierte Phosphorsäure,
0,01 bis 5 Gew.-%, vorzugsweise 0,05 bis 0,5 Gew.-% Korrosionsinhibitor,
0 bis 10 Gew.-%, vorzugsweise 0,5 bis 4 Gew.-% Tensid,
zu 100 Gew.-% Wasser sowie ggf. weitere Hilfsstoffe

Die eigentliche Desinfektion der medizinischen Instrumente wird im erfindungsgemäßen Verfahren dann dadurch vorgenommen, daß diese Instrumente in die aus Schritt B resultierende anwendungsfertige Lösung der peroxidischen Wirkstoffe eingelegt werden, so daß sie vollständig von dieser Lösung benetzt werden. Die Einwirkungszeit hängt von der Konzentration der Desinfektionslösung, aber auch in sehr starkem Maße von dem zu bekämpfenden Keimspektrum ab. Besonders bemerkenswert ist, daß mit dem erfindungsgemäß zu verwendenden Verfahren nicht nur das übliche Keimspektrum an Bakterien und Pilzen beseitigt werden kann, sondern daß auch die besonders widerstandsfähigen Mykobakterien abgetötet werden. Die in Schritt B zur Verfügung gestellte Desinfektionslösung weist eine ausreichend lange Beständigkeit auf und ihre Korrosionswirkung auf metallische Instrumente ist vernachlässigbar klein.

### Beispiele

### 1. Herstellung der Wirkstofflösung

Von einem pulverförmigen Gemisch, bestehend aus
20 Gew.-% Natriumperboratmonohydrat
15 Gew.-% TAED-Pulver
40 Gew.-% Natriumtriphosphat
15 Gew.-% Natriumcarbonat
2 Gew.-% Alkylbenzolsulfonat
8 Gew.-% Natriumsulfat
wurden 2 g in 100 ml Leitungswasser bei Raumtemperatur eingerührt. Nach 15 Min. wurden zu dieser Lösung, die einen pH-Wert von 9,8 aufwies, 0,5 g einer Säuremischung, bestehend aus
60,0 Gew.-% konzentrierter Phosphorsäure
0,2 Gew.-% Octanphosphonsäure
0,4 Gew.-% Fettalkoholethoxylat (Dehypon LS 54® Henkel)
0,4 Gew.-% Natriumcumolsulfonat
39,0 Gew.-% Wasser
hinzugefügt. Die so entstandene Lösung hatte einen pH-Wert von 7,9 und wurde ohne weitere Verdünnung auf ihre Wirkung gegenüber Mykobakterien und auf ihre Korrosionswirkung geprüft.

### 2. Prüfung der Wirksamkeit gegen Mykobakterien

Als Testmethode wurde der "Quantitative Suspensionsversuch mit Mycobakterium terrae für die Prüfung von Instrumentendesinfektionsmitteln" (Hygiene und Medizin 21, 375-376, 1996) verwendet. Bei dieser Methode wird die Abtötung des Testkeimes als logarithmischer Reduktionsfaktor quantitative zur Wasserkontrolle erfaßt.
Geprüft wurde sowohl die nach 1) hergestellte Wirkstofflösung (b) als auch, zum Vergleich eine entsprechende Lösung, deren pH-Wert nicht abgesenkt worden war (a).

| Prüflösungen | Reduktionsfaktor nach Einwirkzeit von | |
|---|---|---|
| | 30 Min. | 60 Min. |
| a) Pulvermischung ohne Säuremischung (2 g in 100 ml Wasser) | 0,14 | 0,38 |
| b) Pulvermischung (2 g in 100 ml Wasser) plus Säuremischung (0,5 in 100 ml Wasser) | 5,07 | ≥5,21 |
| Wasserkontrolle (Lebendkontrolle) | 6,07 | 6,21 |

Bereits nach 30 Minuten wurde mit der erfindungsgemäß zu verwendenden Zubereitung b eine voll ausreichende Wirksamkeit erreicht, während die Referenzlösung a selbst nach 60 Minuten nahezu unwirksam war.

### 3. Prüfung der Korrosionswirkung

Das Korrosionsverhalten der für das erfindungsgemäß zu verwendenden Verfahren bestimmten Desinfektionslösung b wurde untersucht, indem entfettete Eisennägel in die Prüflösung vollständig eingetaucht wurden. Die Auswertung erfolgt nach 8 Stunden im Vergleich mit unbehandelten und in Leitungswasser gelagerten Nägeln. Die Bewertung erfolgte nach visueller Beurteilung von Ablagerungen, Rückständen und sichtbaren Angriffen nach folgender Skala:
0 = keine Korrosion
1 = geringe Korrosion
2 = mittlere Korrosion
3 = starke Korrosion

Geprüft wurden auch hier sowohl die nach 1) hergestellte Wirkstofflösung b als auch im Vergleich, eine entsprechende Lösung a, deren pH-Wert nicht abgesenkt worden war. Als weiterer Vergleich diente das Leitungswasser allein, das zur Herstellung der Prüflösungen verwendet worden war. Es wurden folgende Ergebnisse erhalten:

| Prüflösungen | Bewertung |
|---|---|
| a) Pulvermischung ohne Säuremischung (2 g in 100 ml Wasser) | 0 |
| | |
| b) Pulvermischung (2 g in 100 ml Wasser) plus Säuremischung (0,5 g in 100 ml Wasser) | 0 |
| | |
| Stahdardprobe (Wasser) | 3 |

### 4. Instrumentendesinfektion

Eine Instrumentenwanne wurde mit 20 Liter kaltem Leitungswasser gefüllt. Zur Vorbereitung der Desinfektionslösung wurden gemäß Beispiel 1 zunächst 400 g des pulverförmigen Gemisches eingerührt und nach 15 Minuten 100 g der Säuremischung unter Rühren zudosiert.

Die zu reinigenden und zu desinfizierenden Instrumente wurden in die Desinfektionslösung eingelegt, wobei auf vollständige Benetzung geachtet wurde. Nach einer Einwirkungszeit von 60 Minuten wurden die Instrumente der Lösung entnommen und mit Trinkwasser abgespült.

## Patentansprüche

1. Verwendung eines Desinfektions-Verfahrens für medizinische Instrumente zur Abtötung von Mykobakterien durch Behandeln der Instrumente mit einer mikrobizid wirkenden wäßrigen Zubereitung, **dadurch gekennzeichnet, daß** bei der Herstellung dieser Zubereitung folgende zwei Schritte A und B durchlaufen werden:
A: Umsetzen von Wasserstoffperoxid oder von peroxidischen Verbindungen, die in Wasser Wasserstoffperoxid bilden, in wäßrig-alkalischem Medium bei einem pH-Wert im Bereich von 9 bis 11 mit N-Acylverbindungen, die unter diesen Bedingungen in der Lage sind, Wasserstoffperoxid zu acylieren,
B: Absenken des pH-Wertes der in Schritt A erhaltenen Zubereitung und gegebenenfalls Verdünnung, so daß in der zur Desinfektion eingesetzten Zubereitung ein pH-Wert zwischen 7 und 9 vorliegt.

2. Verwendung nach Anspruch 1, **dadurch gekennzeichnet, daß** die Umsetzung in Schritt A durch Auflösen eines festen Gemisches von anorganischem Peroxid und Acylierungsmittel sowie gegebenenfalls Alkalisierungsmittel und weiteren Hilfsstoffen in Wasser ausgelöst wird.

3. Verwendung nach Anspruch 1 oder 2, **dadurch gekennzeichnet, daß** die in Schritt A eingesetzte peroxidische Verbindung ausgewählt ist aus der Gruppe Natriumperboratmonohydrat, Natriumperborattetrahydrat, Natriumpercarbonat und deren Mischungen.

4. Verwendung nach einem oder mehreren der Ansprüche 1 bis 3, **dadurch gekennzeichnet, daß** das in Schritt A eingesetzte Acytierungsmittel ausgewählt ist aus der Gruppe Tetraacetylglykoluril, Tetraacetylethylendiamin, Diacetylhexahydotriazindion und deren Mischungen.

5. Verwendung nach einem oder mehreren der Ansprüche 1 bis 4, **dadurch gekennzeichnet, daß** in Schritt A in die Zubereitung weitere Hilfsstoffe, vorzugsweise aus der Gruppe Tenside, Alkalisierungsmittel, Komplexbildner für Wasserhärte oder Schwermetallionen, wasserlösliche anorganische Salze und deren Mischungen, eingebracht werden.

6. Verwendung nach einem oder mehreren der Ansprüche 1 bis 5, **dadurch gekennzeichnet, daß** die Absenkung des pH-Wertes in Schritt B durch Zusatz einer Säure aus der Gruppe Phosphorsäure, Essigsäure, Citronensäure und deren Mischungen erreicht wird.

7. Verwendung nach einem oder mehreren der Ansprüche 1 bis 6, **dadurch gekennzeichnet, daß** in Schritt B weitere Hilfsstoffe, vorzugsweise aus der Gruppe Korrosionsinhibitoren, Tenside und deren Mischungen, zugesetzt werden.

8. Verwendung nach einem oder mehreren der Ansprüche 1 bis 7, **dadurch gekennzeichnet, daß** die Umsetzung in Schritt A durch Auflösen von 1 bis 10 Gew.-% einer pulverförmigen Mischung folgender Zusammensetzung
5 bis 40 Gew.-%, vorzugsweise 10 bis 30 Gew.-%, feste anorganische Perverbindung,
5 bis 30 Gew.-%. vorzugsweise 10 bis 20 Gew.-%, pulverförmiges TAED,
20 bis 50 Gew.-%, vorzugsweise 30 bis 45 Gew.-%, Natriumtriphosphat
0 bis 15 Gew.-%, vorzugsweise 1 bis 10 Gew.-%, Tensid und
zu 100 Gew.-% lösliches anorganisches Salz und gegebenenfalls weitere Hilfsstoffe
ausgelöst wird und die Absenkung des pH-Wertes auf pH 7 bis 9 in Schritt B durch Zugabe einer dazu ausreichenden Menge der folgenden Zusammensetzung:
40 bis 80 Gew.-%, vorzugsweise 55 bis 65 Gew.-%, konzentrierte Phosphorsäure
0,01 bis 5 Gew.-%, vorzugsweise 0,05 bis 0,5 Gew.-%, Korrosionsinhibitor,
0 bis 10 Gew.-%, vorzugsweise 0,5 bis 4 Gew.-%, Tensid,
zu 100 Gew.-% Wasser sowie gegebenenfalls weitere Hilfsstoffe
erreicht wird.

## Claims

1. The use of a disinfection process for medical instruments for destroying mycobacteria by treating the instruments with a microbicidally active aqueous preparation, **characterized in that** the following two steps A and B are successively carried out in the production of the microbicidal preparation:
A: reacting hydrogen peroxide or peroxidic compounds which form hydrogen peroxide in water in an aqueous alkaline medium at a pH of 9 to 11 with N-acyl compounds which are capable under these conditions of acylating hydrogen peroxide,
B: reducing the pH value of the preparation obtained in step A and optionally diluting the preparation so that a pH value in the range from 7 to 9 is established in the preparation used for disinfection.

2. The use claimed in claim 1, **characterized in that** the reaction in step A is initiated by dissolving a solid mixture of inorganic peroxide and acylating agent and optionally alkalizing agent and other auxiliaries in water.

3. The use claimed in claim 1 or 2, **characterized in that** the peroxidic compound used in step A is selected from the group consisting of sodium perborate monohydrate, sodium perborate tetrahydrate, sodium percarbonate and mixtures thereof.

4. The use claimed in one or more of claims 1 to 3, **characterized in that** the acylating agent used in step A is selected from the group consisting of tetraacetyl glycol uril, tetraacetyl ethylenediamine, diacetyl hexahydrotriazine dione and mixtures thereof.

5. The use claimed in one or more of claims 1 to 4, **characterized in that**, in step A, other auxiliaries, preferably from the group consisting of surfactants, alkalizing agents, complexing agents for water hardness or heavy metal ions, water-soluble inorganic salts and mixtures thereof, are introduced into the preparation.

6. The use claimed in one or more of claims 1 to 5, **characterized in that** the lowering of the pH value in step B is achieved by adding an acid from the group consisting of phosphoric acid, acetic acid, citric acid and mixtures thereof.

7. The use claimed in one or more of claims 1 to 6, **characterized in that** other auxiliaries, preferably from the group consisting of corrosion inhibitors, surfactants and mixtures thereof, are added in step B.

8. The use claimed in one or more of claims 1 to 7, **characterized in that** the reaction in step A is initiated by dissolving 1 to 10% by weight of a powder-form mixture with the following composition:
5 to 40% by weight, preferably 10 to 30% by weight of solid inorganic per compound,
5 to 30% by weight, preferably 10 to 20% by weight of powder-form TAED,
20 to 50% by weight, preferably 30 to 45% by weight of sodium triphosphate,
0 to 15% by weight, preferably 1 to 10% by weight of surfactant and balance to 100% by weight soluble inorganic salt and optionally other auxiliaries,
and the lowering of the pH value to pH 7.5-8.5 in step B is achieved by adding an adequate quantity of the following composition:
40 to 80% by weight, preferably 55 to 65% by weight of concentrated phosphoric acid,
0.01 to 5% by weight, preferably 0.05 to 0.5% by weight of corrosion inhibitor,
0 to 10% by weight, preferably 0.5 to 4% by weight of surfactant, balance to 100% by weight water and optionally other auxiliaries.

## Revendications

1. Utilisation d'un procédé de désinfection pour instruments médicaux en vue de la destruction de mycobactéries, par traitement des instruments avec une préparation aqueuse à action microbicide,
**caractérisée en ce que**
dans la production de cette préparation, deux étapes suivantes A et B sont parcourues :
A) conversion du peroxyde d'hydrogène ou de composés peroxydiques qui forment dans l'eau du peroxyde d'hydrogène, en un milieu hydro-alcalin, à une valeur de pH dans la zone de 9 à 11 avec des composés N-acylés qui sont en mesure dans ces conditions, d'acyler le peroxyde d'hydrogène,
B) abaissement de la valeur du pH de la préparation obtenue à l'étape A et éventuellement dilution de façon que la préparation mise en oeuvre pour la désinfection ait une valeur de pH comprise entre 7 et 9.

2. Utilisation selon la revendication 1,
**caractérisée en ce que**
la réaction à l'étape A est déclenchée par mise en solution d'un mélange solide de peroxyde minéral et d'agent d'acylation ainsi que le cas échéant un agent d'alcalinisation et d'autres adjuvants, dans l'eau.

3. Utilisation selon la revendication 1 ou la revendication 2,
**caractérisée en ce que**
le composé peroxydique mis en oeuvre à l'étape A, est choisi dans le groupe du monohydrate de perborate de sodium, du tétrahydrate de perborate de sodium, du percarbonate de sodium et leurs mélanges.

4. Utilisation selon l'une ou plusieurs des revendications 1 à 3,
**caractérisée en ce que**
l'agent d'acylation mis en oeuvre dans l'étape A, est choisi dans le groupe du tétraacétylglycolurile, de la tétracétyléthylènediamine, de la diacétylhexahydrotriazinedione, et de leurs mélanges.

5. Utilisation selon l'une ou plusieurs des revendications 1 à 4,
**caractérisée en ce que**
dans l'étape A on introduit dans la préparation, d'autres substances auxiliaires choisies de préférence dans le groupe des agents tensioactifs, des agents d'alcalinisation, des agents complexants pour la dureté de l'eau ou les métaux lourds, des sels minéraux solubles dans l'eau, et de leurs mélanges.

6. Utilisation selon l'une ou plusieurs des revendications 1 à 5,
**caractérisée en ce que**
l'abaissement de la valeur du pH à l'étape B est obtenu par addition d'un acide choisi dans le groupe de l'acide phosphorique, l'acide acétique, de l'acide citrique et de leurs mélanges.

7. Utilisation selon l'une ou plusieurs des revendications 1 à 6,
**caractérisée en ce qu'**
à l'étape B on ajoute d'autres substances auxiliaires de préférence choisies dans le groupe des inhibiteurs de corrosion, des agents tensioactifs et de leurs mélanges.

8. Utilisation selon l'une ou plusieurs des revendications 1 à 7,
**caractérisée en ce que**
la réaction à l'étape A est déclenchée par mise en solution de 1 à 10 % en poids d'un mélange pulvérulent ayant la composition suivante :
- de 5 à 40 % en poids, de préférence de 10 à 30 % en poids de composé peroxydique solide minéral,
- de 5 à 30 % en poids, de préférence de 10 à 20 % en poids de TAED pulvérulent,
- de 20 à 50 % en poids, de préférence de 30 à 45 % en poids, de triphosphate de sodium,
- de 0 à 15 % en poids, de préférence de 1 à 10 % en poids, d'agent tensioactif, et
pour 100 % en poids un sel minéral soluble et le cas échéant d'autres substances auxiliaires, et
l'abaissement de la valeur du pH à pH 7 à 9 à l'étape B, est obtenu par addition d'une quantité suffisante pour cela de la composition suivante :
- de 40 à 80 % en poids, de préférence de 55 à 65 % en poids, d'acide phosphorique concentré,
- de 0,01 à 5 % en poids, de préférence de 0,05 à 0,5 % en poids d'inhibiteur de corrosion,
- de 0 à 10 % en poids, de préférence de 0,5 à 4 % en poids d'agent tensioactif,
pour 100 % en poids de l'eau ainsi qu'éventuellement d'autres agents auxiliaires.
